Europäisches Patentamt

European Patent Office

Office européen des brevets

(11) Publication number: **0 517 154 A1**

## EUROPEAN PATENT APPLICATION

(21) Application number: **92109271.4**

(22) Date of filing: **02.06.92**

(51) Int. Cl.5: **C12Q 1/68**, C12P 19/34,
C07H 21/04

(30) Priority: **06.06.91 US 712904**

(43) Date of publication of application:
**09.12.92 Bulletin 92/50**

(84) Designated Contracting States:
**AT BE CH DE DK ES FR GB GR IT LI LU NL PT SE**

(71) Applicant: **F. HOFFMANN-LA ROCHE AG**
**Grenzacherstrasse 124**
**CH-4002 Basel(CH)**

(72) Inventor: **Atlas, Ronald M.**
**1603 Dunbarton Wynde**
**Louisville, Kentucky 40205(US)**
Inventor: **Bej, Asim K.**
**778 David Fairleigh Court**
**Louisville, Kentucky 40217(US)**
Inventor: **Mahbubani, Meena H.**
**778 David Fairleigh Court**
**Louisville, Kentucky 40217(US)**

(74) Representative: **Notegen, Eric-André et al**
**Grenzacherstrasse 124 Postfach 3255**
**CH-4002 Basel(CH)**

(54) **Method for detecting giardia.**

(57) This invention provides for improved methods for detecting microorganisms, especially species of *Giardia*, in water and other samples. The methods rely on nucleic acid probes and amplification primers specific for subsequences of the gene encoding *giardin*. This invention also provides for primers and probes specific for amplifying and detecting subsequences of *giardin* in samples where the subsequences are either conserved across the genera or specific to human pathogenic forms of *Giardia*. In addition, this invention provides for an effective means of distinguishing between living and dead waterborne organisms such as *Giardia* cysts based upon the presence or absence of heat-induced mRNA. Finally this invention provides for kits embracing the above aspects.

EP 0 517 154 A1

This invention provides for methods for detecting species of *Giardia* in water and other samples and for primers and probes specific for amplifying and detecting subsequences of *giardin* in samples where the subsequences are either conserved across the genera, or specific to human pathogenic forms of *Giardia*. In addition, this invention provides for an effective means of distinguishing between living and dead waterborne organisms such as *Giardia* cysts and for kits embracing the above aspects.

This invention provides for a method for detection of species of the genus *Giardia* having a copy of the gene encoding *giardin*, said method comprising: (a) hybridizing a nucleic acid probe to a complementary portion of the gene encoding *giardin*; and, (b) detecting the hybridization of the probe to the gene encoding *giardin*. More preferred is the above method which further comprises before step (a), the step of amplifying a subsequence of the *giardin* gene. The polymerase chain reaction method is a preferred means for amplifying the subsequence.The probes may be designed to bind to a conserved region or a hypervariable region of the gene encoding *giardin*. There are disclosed herein probes which bind to specific subsequences of the gene encoding *giardin*.

The use of an amplification method involving nucleotide polymerases for amplification of nucleic acid subsequences from a *Giardia* species is also disclosed. Specific subsequences to which amplification primers bind and specific primers are also disclosed. The binding regions, to which primers bind, flank target subsequences of the gene encoding *giarain*.

In addition to the above methods, nucleic acid probes and primers for use in the defined methods are also disclosed.

Kits are also disclosed for the detection of nucleic acid specific for *Giardia* species comprising a compartment which contains a nucleic acid probe which binds substantially to a nucleic acid subsequence of the gene encoding *giardin*.More particularly the kit comprises the primers and probes described herein.

This invention further discloses a method of distinguishing live organisms from dead organisms in a sample suspected of containing living organisms. The method comprises: (a) treating the sample with sufficient heat to induce transcription of genes encoding proteins not otherwise transcribed; (b) lysing the cysts to release mRNA; (c) reverse transcribing the mRNA to cDNA; (d) amplifying target subsequences of the mRNA encoding the heat-induced genes; and (e) detecting the amplified target subsequences. More particularly, the genes are selected from the group consisting of heat shock genes and *giardin*. For example, the live organisms may be cysts originating from species of *Giardia* and the method further comprises: (a) treating the cysts to heat sufficient to induce transcription of the gene encoding *giardin*; (b) lysing the cysts to release mRNA; (c) reverse transcribing the mRNA to cDNA; (d) amplifying target subsequences of the mRNA encoding the *giardin* gene; and (e) detecting the amplified target subsequences. Amplification is preferably done using the amplification primers described herein.

In addition to kits, amplification tubes containing the necessary amplification reagents to amplify the described target subsequences for *Giardia* species are described herein.

"Amplification reaction mixture" refers to an aqueous solution comprising the various reagents used to amplify a target nucleic acid. These include: enzymes, aqueous buffers, salts, target nucleic acid, and nucleoside triphosphates. Depending upon the context, the mixture can be either a complete or incomplete amplification reaction mixture.

"Amplification reaction system" refers to any *in vitro* means for multiplying the copies of a target sequence of nucleic acid. Such methods include, but are not limited to, polymerase chain reaction amplification [PCR], DNA ligase, QB RNA replicase, and RNA transcription-based amplification systems. These involve multiple amplification reagents and are more fully described below.

"Amplification reaction tube(s)" refers to a container suitable for holding the amplification reagents. Generally, the tube is constructed of inert components so as to not inhibit or interfere with the amplification system being used. Where the system requires thermal cycling of repeated heating and cooling, the tube must be able to withstand the cycling process and typically precisely fit the wells of the thermocycler.

"Amplification reagents" refer to the various buffers, enzymes, primers, nucleoside triphosphates (both conventional and unconventional), and probes used to perform the selected amplification procedure.

"Amplifying" or "Amplification", which typically refers to an "exponential" increase in target nucleic acid, is being used herein to describe both linear and exponential increases in the numbers of a select target sequence of nucleic acid.

"Bind(s) substantially" refers to complementary hybridization between oligonucleotides and embraces minor mismatches which can be accommodated by reducing the stringency of the hybridization media to achieve the desired priming of the PCR polymerases.

"Conserved region(s)" refers to subsequences of a genome which are present in the genomes of most, if not all, species of the same genera.

"Hybridizing" refers the binding of two single stranded nucleic acids via complementary base pairing.

"Hypervariable region(s)" refers to subsequences of a genome which are present in the genomes of one or a very few of the species of a genera (i.e., less than 30% of the species).

"Internal positive control (IPC) oligonucleotide sequence" refers to a recombinant or synthetic oligonucleotide that amplifies with the same primer pair used to amplify target nucleic acid. IPC oligonucleotide sequences are used to ensure assay users that the amplification process has occurred in the event that the sample being tested has no target nucleic acid. These oligonucleotides are flanked at one or both ends by a sequence complementary to the binding site of an amplification primer used in the amplification process. The internal target subsequence is a foreign sequence not naturally found adjacent to the binding sites of the amplification primers. The internal control oligonucleotide sequences thus serve as templates for the amplification primers to establish that the amplification reaction would have amplified target nucleic acid if such target had been present. IPC oligonucleotide sequences can be supplied as single or double stranded nucleotides. When single stranded, those of skill would recognize that to facilitate non-linear amplification, the 5' end of the IPC should have base identity with the other primer of the pair.

"*Giardia* species" are members of the family Hexamitidae. It should be recognized that the taxonomy of prokaryotes is not static and that additional species might be named or that these species may some day be incorporated into an alternative family or genus.

"Nucleic acid" refers to a deoxyribonucleotide or ribonucleotide polymer in either single- or double-stranded form, and unless otherwise limited, would encompass known analogs of natural nucleotides which can function in a similar manner as naturally occurring nucleotides.

"Nucleotide polymerases" refers to enzymes able to catalyze the synthesis of DNA or RNA from nucleoside triphosphate precursors. In the amplification reactions of this invention, the polymerases are template dependent and typically extend from the 3' end of the polymer being formed. It is most preferred that the polymerase is thermostable as described in U.S. Patent No.4,889,819.

"Primer" or "nucleic acid polymerase primer(s)" refers to an oligonucleotide, whether natural or synthetic, capable of acting as a point of initiation of DNA synthesis under conditions in which synthesis of a primer extension product complementary to a nucleic acid strand is initiated, i.e., in the presence of four different nucleotide triphosphates and an agent for polymerization (i.e., DNA polymerase or reverse transcriptase) in an appropriate buffer and at a suitable temperature. A primer is preferably an oligodeoxyribonucleotide and is single stranded for maximum efficiency in amplification, but may also be double stranded. If double stranded, the primer is first treated to separate its strands before being used to prepare extension products. The exact length of a primer will depend on many factors, but typically ranges from 15 to 25 nucleotides. Short primer molecules generally require cooler temperatures to form sufficiently stable hybrid complexes with the template. A primer need not reflect the exact sequence of the template, but must be sufficiently complementary to hybridize with a template. An example of a non-complementary sequence which may be incorporated into the primer is a sequence which encodes a restriction enzyme recognition site (see U.S. Patent No. 4,800,159).

A primer can be labeled, if desired, by incorporating a label detectable by spectroscopic, photochemical, biochemical, immunochemical, or chemical means. For example, useful labels include $^{32}$P, fluorescent dyes, electron-dense reagents, enzymes, biotin, or haptens or proteins for which antisera or monoclonal antibodies are available. A label can also be used to "capture" the primer so as to facilitate the immobilization of either the primer or amplified DNA on a solid support.

"Probe" refers to an oligonucleotide which binds through complementary base pairing to a subsequence of a target nucleic acid. The probes are preferably directly labelled as with isotopes or indirectly labelled such as with biotin to which a streptavidin complex may later bind. By assaying for the presence or absence of the probe, one can detect the presence or absence of the target.

"Recombinant" when referring to a nucleic acid probe refers to an oligonucleotide which is free of native proteins and nucleic acid typically associated with probes isolated from the cell which naturally contains the probe sequence as a part of its native genome. Recombinant probes include those made by amplification means such as PCR and genetic cloning methods where bacterium are transformed with the recombinant probe.

"Subsequence" refers to a sequence of nucleic acids which comprise a part of a longer sequence of nucleic acids.

"Target region" refers to a subsequence of a nucleic acid which is to be analyzed, which usually contains polymorphic DNA sequences.

This invention provides improved means for the detection of *Giardia* species using the gene encoding *giardin* as the target for detection. The various embodiments of this invention include nucleic acid hybridization assays wherein the nucleic acid probes bind to target sequences of the *giardin* gene and PCR amplification of select target subsequences of the *giardin* gene prior to detection. In addition, it is disclosed herein that the *giardin* gene can be heat-induced to transcribe in cysts. This fact is advantageously used to distinguish between living and dead cysts in samples.

The *giardin* gene has several conserved regions. They are between 78 to 190 bp, 267 to 385 bp and 639 to 809 bp. The gene also has hypervariable regions. They are located between 405 to 622 bp. The *giardin* gene is a definitive taxonomic determinant for the *Giardia* genus. The conserved and hypervariable regions of the gene can be used as targets for detection assays based upon nucleic acid hybridization. Probes which bind substantially to the conserved regions will permit detection of all members of the genus. Probes which bind substantially to the hypervariable regions will permit detection of subgroups within the genus or to specific species.

Nucleic acid hybridization technology is well known. Many commercial kits are presently available which utilize nucleic acid hybridization to detect microorganisms. A good review of the technology may be found in *Nucleic Acid Hybridization*, A *Practical Approach*, Ed Hames and Higgins, IRL Press. pp. 18-30, 1985 or in U.S. Patent No. 4,886,741, columns 4-7. In view of the routine nature of the technology, no attempt will be made herein to provide an overview of this technology.

It is helpful to amplify select subsequences of the *giardin* gene prior to detection. A number of nucleic acid amplification systems are available. Amplification systems are well known and have been reviewed in *Bio/Technology* 8:290-293, 1990. Such systems include PCR, ligase amplification and the Q$\beta$ replication system.

The detection of *Giardia* species preferably takes advantage of PCR to amplify nucleic acid from these species. The detection of *Giardia* as disclosed herein requires multiple steps. These steps include an adequate sampling procedure to isolate and/or concentrate the extant organisms to a suitable degree for detection, a method for lysing the cells to release nucleic acid, a suitable procedure to amplify the target nucleic acid sequences, and a means to detect the amplified sequences. A general description of this methodology can be found in Atlas, R.M. and Bej, A.K., Detecting Bacterial Pathogens in Environmental Water Samples by Using PCR and Gene Probes in PCR Protocols, pp. 399-406, Ed. Innis et al., Academic Press, Inc., 1990.

To effectively amplify target nucleic acid subsequences, PCR requires amplification primers to initiate polymerase extension. The selection of primers is an important aspect of this invention. The primers are first selected for their ability to bind to flanking regions of conserved or hypervariable regions. The primers are further selected for low secondary structure and relatively similar thermal melting points.

Relatively clean water samples may be directly assayed without isolation or purification of the target nucleic acid prior to amplification. Large water samples may be filtered to concentrate microbial populations. Filtering procedures and filters are well known to those of skill. See, for example, *Standard Method For The Examination Of Water* and *Wastewater*, 17th Ed., pp. 9-14, published by the Amer. Publ. Health Assoc., 1015 Fifteenth St. N.W., Washington, D.C. 20005. Preferred filters have a porosity which will trap *Giardia* cysts yet permit extraneous material to pass.

It is preferred that substantially all the cells or cysts of the targeted waterborne pathogen from a water sample are concentrated into a small test sample volume of water on the order of about 0.1 to about 1.0 ml. With respect to the *Giardia* cysts, the operation of recovering substantially all the targeted cysts from a water sample may be performed by any of several suitable means, including, for example, filtration and centrifugation, possibly with the help of suspended or dissolved additives which service to capture or flocculate the target organisms in a physical state which facilitates their separation. Preferred cyst recovery according to this invention is obtained by centrifugation from small volume samples, about 1 mL or less, or by filtration from 100 ml samples that may have been concentrated and partially purified from much larger volumes.

*Giardia* cysts can be trapped by filters having a porosity of about 0.5 $\mu$m or less. A prefilteration step can be useful where physical debris may interfere with the subsequent filtration or amplification procedures. The prefilter is a noncritical feature of this invention. Such filters are generally of a porosity and composition that will allow the *Giardia* bacterium to pass freely through it. The physical means for water collection are well known and include sterile glass vials, syringe assemblies, and other inert vessels suitable for storage of water.

Teflon membranes such as Fluoropore FGLP 0013 of 0.2 $\mu$M, or 0.5 $\mu$M pore size (Millipore Corp., Bedford, MD), are of use in this invention. Teflon membranes do not interfere with the PCR amplification reaction and may be left in the amplification reaction mixture.

The *Giardia* cysts are lysed in a manner which releases essentially all target DNA or RNA. The target nucleic acid is then recovered from the cysts so as to be sufficiently free of potentially interfering substances, such as enzymes, low molecular weight inhibitors, or other components that might interfere with enzymatic amplification of the target DNA sequences.

Mechanical lysis is useful. Mechanical lysis can be achieved by sonication or multiple freeze/thaw cycles. Cycles of freezing and thawing are more preferred. Chemical means of cell disruption are also operable and include standard lysing means such as lysozymes, osmotic shock, protease K treatment, and detergents. Chemical methods are less preferred because of possible detrimental effects on the PCR process, i.e. inhibition of the Taq polymerase.

The samples can also be heated to denature proteases and nucleases which might interfere with the components of the PCR reaction mixture. Chemical nuclease and protease inhibitors can also be used in combination with heat. A preferred means for lysing the cysts is performed by heating the cysts to 90°C for 5 mins in 50 $\mu$l of 0.1% diethylpyrocarbonate (DEPC) in water in the thermal cycler, followed by quick cooling in ice or quick cooling the cells to 5°C for 2 mins in the thermal cycler. Alternately, nucleic acid may be released from cysts by freeze-thaw cycling.

In the case of RNA detection, DNA is destroyed by deoxyribonuclease, cDNA is reverse-transcribed and then target DNA is amplified using PCR. Following PCR amplification of targeted nucleic acid sequences, amplified targeted DNA is detected by sufficiently sensitive and specific detection methods. Detection is by means of suitable hybridization probes utilizing probes of specific DNA sequences from each of the targeted gene sequences. Quantification of the amplified target DNA sequences may also be carried out, if desired.

When the sample is a complex mixture such as a fecal sample from a patient suspected of being infected with *Giardia*, it may be necessary to isolate the nucleic acid from that complex mixture. A variety of techniques for extracting nucleic acids from biological samples are known in the art. For example, see those described in Higuchi, "Simple and Rapid Preparation of Samples for PCR" Chapter 4 in PCR *Technology* ed. Erlich, Stockton Press 1989; Maniatis et al., 1989, *Molecular Cloning: A Laboratory Manual*, New York, Cold Spring Harbor Laboratory, 1982; Hagelberg and Sykes, 1989, *Nature* 342:485; or Arrand, Preparation of Nucleic Acid Probes in *Nucleic Acid Hybridization, A Practical Approach*, Ed Hames and Higgins, IRL Press. pp. 18-30, 1985. Whole nucleic acid extraction procedures typically involve an initial contacting with phenol, phenol/chloroform or guanidinium salts, followed by an alcohol precipitation. Genomic DNA may be obtained from a whole nucleic acid extraction by using RNase before further alcohol precipitation.

Although the PCR process is well known in the art (see U.S. Patent Nos. 4,683,195 and 4,683,202 some general PCR information is provided below for purposes of clarity and full understanding of the invention to those unfamiliar with the PCR process.

To begin the PCR process, the target nucleic acid in the sample is denatured (assuming the sample nucleic acid is double-stranded). Denaturation is typically achieved by heating the samples. This is because chemical denaturants may inhibit the polymerase activity.

Once the strands are separated, the next step in PCR involves hybridizing the separated strands with primers that flank the target region or subsequence. The primers are then extended to form complementary copies of the target strands, and the cycle of denaturation, hybridization, and extension is repeated as many times as necessary to obtain the desired amount of amplified nucleic acid.

Template-dependent extension of primers in PCR is catalyzed by a polymerizing agent in the presence of adequate amounts of four deoxyribonucleotide triphosphates (dATP, dGTP, dCTP, and dTTP) in a reaction medium comprised of the appropriate salts, metal cations, and pH buffering system. Suitable polymerizing agents are enzymes known to catalyze template-dependent DNA synthesis. For example, if the template is RNA, a suitable polymerizing agent to convert the RNA into a complementary DNA (cDNA) sequence is reverse transcriptase (RT), such as avian myeloblastosis virus RT. Once the target for amplification is DNA, suitable polymerases include, for example, *E. coli* DNA polymerase I, or its Klenow fragment, $T_4$ DNA polymerase, and *Taq* polymerase, a heat stable DNA polymerase isolated from *Thermus aquaticus* and commercially available. The latter enzyme, *Taq* DNA polymerase, is widely used in the amplification and sequencing of nucleic acids. The reaction conditions for using DNA polymerases are known in the art, and are described in, for example, the treatise *Methods in Enzymology*, and in Maniatis *et al.*, *Molecular Cloning: A Laboratory Manual, supra*.

During the PCR process, the temperature of the amplification reaction mixture is very carefully controlled so that strand separation and primer annealing and extension occur in equilibrium. The reaction mixture is repeatedly cycled between (1) a low temperature, generally of from about 37°C to 70°C, for primer annealing to the selected target sequence for strand reassociation, (2) an intermediate temperature, generally of from about 70°C to 80°C, for polymerase extension of the primers, and (3) a higher temperature, generally of from about 80°C to 100°C, for denaturation or separation of the strands. Although three temperature ranges have been described, it is often possible that the amplification process can be adequately conducted between two of the temperature ranges. Each thermal cycle of the two or three temperatures can increase the concentration of the amplified target DNA sequence as much as two-fold, so that every series of ten amplification cycles can increase the concentration as much as 1024-fold.

In the preferred embodiment of the PCR process, the reaction is catalyzed by a thermostable DNA polymerase enzyme. The preferred thermostable polymerase is the *Taq* DNA polymerase purified from the bacterium *Thermus aquaticus*. The PCR reaction is carried out at an elevated temperature. The preferred temperature is one at which the enzyme is thermostable, and at which the nucleic acids are in an equilibrium of single and double strands, so that sufficient primer will anneal to template strands to allow a reasonable rate of polymerization. Strand separation is achieved by heating the reaction to a sufficiently high temperature for sufficient time to cause the denaturation of the duplex, but not to cause an irreversible denaturation of the polymerase. If *Taq* is used, the polymerase reaction can be cycled many times, typically 20-40 times, between the two or three temperatures without need to augment the initially added polymerase enzyme.

The PCR method can be performed in a step-wise fashion, where after each step new reagents are added, or in a fashion where all of the reagents are added after a given number of steps. For example, if strand separation is induced by heat, and the polymerase is heat-sensitive, then the polymerase will have to be added after every round of strand separation. However, if, for example, a helicase is used for denaturation, or if a thermostable polymerase is used for extension, then all of the reagents may be added initially; or, alternatively, if molar ratios of reagents are of consequence to the reaction, the reagents may be replenished periodically as they are depleted by the synthetic reaction.

Those skilled in the art will know that the PCR process is generally carried out as an automated process with a thermostable enzyme. In this process, the reaction mixture is cycled through a denaturing step, a primer annealing step, and an extension step. A DNA thermocycler, a machine specifically adapted for use with a thermostable enzyme, is disclosed more completely in EP 236,069 and U.S. Patent No. 4,889,819.

A preferred mode for carrying out the PCR reaction is the multiplex mode. The multiplex mode involves the simultaneous amplification of different target regions using more than one set of PCR primer pairs. The multiplex procedure is preferably designed around primer pairs which have similar thermal melting points. It is preferred that all pairs have thermal melting points within 8°C of each other and that the average thermal melting point is between 45°C and about 70°C with preference for an average thermal melting point of between 60°C and 70°C.

More particularly, after recovery of substantially undegraded target DNA in a small volume of about 50 $\mu$l of water or aqueous buffer, and after selection of the appropriate oligonucleotide primer pair for the targeted DNA sequence, the target DNA is incubated with dNTP's, Mg+2, a DNA polymerase and the oligonucleotide primers under conditions where the primers hybridize to the separated (denatured) target DNA strands and the polymerase extends the primers to make fully double-stranded replicas of the target sequence.

The PCR amplification conditions, such as temperatures, incubation times, solvents, enzyme choice, reagent concentrations, equipment and the like, are chosen to give efficient and specific amplification of the target DNA sequence. It will be readily understood that the effective and optional conditions for each process step and parameter will differ significantly between the target organisms, the various kinds of test samples, target DNA sequence and primer pairs selected. Solvent choice, enzyme choice and concentration, primer concentration, dNTP concentration, and equipment choice for performing thermal cycles with sufficiently well controlled temperatures and incubation times are generally understood by those skilled in the art of PCR amplification of DNA.

Choice of optimum temperatures and incubation times for the specific target sequences of the invention may be determined by routine titration, monitoring the quantity and quality of amplified DNA, such as by agarose or polyacrylamide gel electrophoresis after staining of DNA with a fluorescent dye such as ethidium bromide. One selects reaction conditions which maximize the yield of an electrophoretic band of target DNA with the size expected to be defined by the chosen primers and minimize, or preferably completely prevent, amplification of any other DNA.

As examples of specifically preferred conditions for conducting PCR DNA amplification of this invention, there may be mentioned: initial denaturation at 94°C for 3 min; reannealing and extension temperature of 50°C for 1 min; denaturation temperature of 94°C for 1 min, and magnesium concentration 1.5 mM for GGL78-97 and GGR171-190; and GGL267-286 and GGR366-385 and GGL405-433 and GGR592-622; initial denaturation of 94°C for 3 min, reannealing temperature of 60°C for 60 sec; denaturation temperature of 94°C for 60 sec; and magnesium concentration of 1.5 mM for GGL639-658 and GGR789-809. The base sequence for the primers are provided in Table2 below.

For PCR amplification starting with RNA, undegraded target RNA is recovered from cysts in 50 $\mu$l 0.1%

DEPC containing water by heating to 90°C for 5 min, followed by cooling to 5°C for 2 min. DNA is degraded by treatment with deoxyribonuclease at 37°C for 60 min. cDNA is reverse transcribed from target mRNA using Murine Moloney Leukemia Virus (MMLV) Reverse Transcriptase and primer GGR789-809. Then PCR amplification is performed as described above.

The gene encoding *giardin* is sufficiently conserved among the *Giardia* genus to permit its use as a target in detection assays. Although the *giardin* gene can serve as the target sequence without amplification, amplification of select nucleic acid subsequences for target regions can significantly enhance the sensitivity of the detection assays. Often subsequences within a gene differ widely in polymorphism, a fact that can be used to advantage in environmental *Giardia* monitoring. One of the significant discoveries of the disclosed invention is the identification of hypervariable regions of the *giardin* gene which permit the assays to differentiate between human pathogenic *Giardia* and harmless species.

Although the nucleotide sequence of the *giardin* gene was previously known, the potential of using the gene as a target for detecting *Giardia,* either directly or after PCR amplification, was not recognized nor was it apparent that it would provide a basis for doing so prior to this invention.

This invention includes the following new and novel discoveries: (1) the *giardin* gene contains 3 sufficiently conserved regions to permit PCR amplification and gene probe detection not only of the human pathogenic *Giardia* strains but of other *Giardia* species such as: *G. lamblia* (= *G. intestinalis*), *G. duodenalis* and *G. muris*, and that this conserved region does not occur in other protozoa, algae, bacteria, fungi and human DNA; and, (2) the *giardin* gene contains a sufficiently hypervariable region to permit PCR amplification and gene probe detection of only the *G. duodenalis* group that includes the human pathogen *G. lamblia*, but not the nonhuman pathogenic species *G. muris* and *G. ardeae*. Moreover, this hypervariable region does not occur in other protozoa, algae, bacteria, fungi and human DNA -- hence, this *giardin* sequence is a suitable target for detecting the human pathogenic strains of *Giardia*.

Specifically, preferred gene subsequences for use in the process of this invention are defined by the primer pairs provided in Table 1. Four separate regions of the *giardin* gene *G. lamblia* are amplified by the four amplification primer pairs. The position numbers for the *giardin* sequence are based on those assigned in the NIH-BIONET data bank. The sequence of the *giardin* gene is publicly available and was published by Baker et al., *Nucl. Acid. Res*. 16:7177, 1988. The sequence is incorporated by reference herein. By convention, the upstream or left primers (GGL) are identical to the published subsequence and the downstream or right are complementary to the published subsequence.

Table 1. Amplification Primers

1. GGL78-97        5'-AAGCTCAGCAACATGAACCA-3' (SEQ ID NO 17), and

2. GGR171-190      5'-ATGGCGTCCTTGATCATCTT-3' (SEQ ID NO 18), or

3. GGL267-286      5'AAGAAGTCCGCCGACAACAT-3' (SEQ ID NO 19), and

4. GGR366-385      5'-AGATTTGTCTCAACGTTGTT-3' (SEQ ID NO 20), or

5. GGL639-658      5'-AAGTGCGTCAACGAGCAGCT-3' (SEQ ID NO 21), and

6. GGR789-809      5'-TTAGTGCTTTGTGACCATCGA-3' (SEQ ID NO 22) for genus *Giardia*

7. GGL405-433      5'-CATAACGACGCCATCGCGGCTCTCAGGAA-3' (SEQ ID NO 23), and

8. GGR592-622      5'-TTTGTGAGCGCTTCTGTCGTGGCAGCGCTAA-3' (SEQ ID NO 24) for *G. lamblia and duodenalis*

Effective primers may also be constructed not only from these subsequences, but from sequences which are contained within them, sequences which overlap them substantially, that is, by approximately 10 bp, sequences within the target gene, and sequences that encompass the target gene.

An internal positive control sequence (IPC) may also be included to ensure that the PCR procedure functions properly. The IPC comprises an amplifiable target which acts as a positive control regardless of whether the *Giardia* target nucleic acid is present. In a preferred embodiment, the IPC comprises an unnatural sequence selected from a portion of the human HLA DQα gene. The gene sequences are flanked by sequences complementary to the upstream and downstream primers used to amplify subsequences of either the target sequences. Human HLA DQα is a sequence which is relatively rare in water samples and not likely to be present in the sample at a detectable level. The sequence for a preferred IPC is provided in Table 2. The IPC can be either double or single stranded and may be stored in the amplification reaction mixture or in a separate container. Typically, IPC is added to an amplification reaction mixture at 1 million to 10 million copies. For quantitation of *Giardia* present in the samples, one can use lower amounts of IPC, e.g., 50 to 100 copies. A 25 mM $MgCl_2$ solution is included to start the reaction by activation of Taq polymerase.

Table 2.  Internal Positive Control Sequence (IPC) and
          Probes.


     5'-UPSTREAM PRIMER BINDING SITE-
TTTGATGGAGATGAGGAGTTCTACG

                                        ...1.2, 1.3,
4......>


     TGGACCTGGAGAGGAAGGAGACTGCCTGGCGGTGGCCTGAGTTCAGCA
..

                                        ...1 probe


AATTTGGAGGTTTTG-DOWNSTREAM PRIMER BINDING SITE-3'
.........


     Positive Control - DQα1
          5'-TGAGTTCAGCAAATTTGGAG-3'


     Negative Control - DQα 1.2, 1.3, 4
               *
          5'-GATGAGCAGTTCTACGTGG-3'


     *  represents a single mismatched base pair.


The methods for the detection of a non-amplified target gene subsequence and an amplified target gene subsequence are essentially the same. The only distinction is that the non-amplified target is generally not available in sufficient amounts to permit the use of less sensitive detection means. For example, the detection of the target sequences can be accomplished by direct visualization of the gels following ethidium bromide staining or by indirect means using specific nucleic acid hybridization probes. However, the indirect means are more sensitive and are therefore recommended for both amplified and non-amplified targets. These methods are well known to those of skill and a general review of such techniques can be found in *Nucleic Acid Hybridization, A Practical Approach*, Eds. Hames and Higgins, IRL Press, Washington, D.C., 1985.

When detection of the target gene subsequence is by hybridization as in a Southern blot, nucleic acid probes specifically complementary to a subsequence of the amplified region are used. Such probes are readily obtainable from the sequence of the amplified segment. Probes preferably hybridize to a DNA subsequence located between the primer binding subsequences to avoid any overlap of primer sequences and probe sequence. The preferred probes for use with the primers given in Table 1 are provided in Table 3. Those of skill will readily recognize that alternative probes altered in length and in binding sites could be used.

Depending on the assay format, it may be helpful to have labelled probes function to detect the amplified product. The probes can be labeled by any of the methods known in the art. Radioactive and enzyme labels are preferred. Most preferred are enzyme labels, which in the presence of an appropriate substrate, will produce a colored product, examples include horseradish peroxidase and alkaline phosphatase. Color development can be accomplished by a variety of means using a variety of known substrates. Preferred methods for horseradish peroxidase include using tetramethylbenzidine (TMB) as described in *Clin. Chem.* 33(8):1368-1371, 1987. An alternative detection system is the Enhanced Chemiluminescent (ECL) detection kit commercially available.

The electrophoresing conditions and the means for detecting the individual amplified oligonucleotides are well known and are not critical aspects of this invention. Any of the means accepted by those of skill will be applicable for this invention.

An alternative mode of detection of the amplified product is by sandwich hybridization onto membrane strips. In the typical arrangement, the strip is made of nylon and has oligonucleotide capture probes covalently bound in discrete spots. Capture probes and detection probes are essentially the same as they both function by binding to complementary subsequences of a target region. Thus the probes of Table3 may function as capture probes.

To bind oligonucleotide capture probes to nylon, they are first tailed with oligothymidylic acid of about 100 bases. Tailing can be achieved using terminal transferase, such as calf-thymus terminal deoxynucleotidyl transferase, or polythymidine oligonucleotides can be chemically synthesized along with the probes. The tailed oligonucleotides are spotted onto the nylon strips. The strips are dried overnight and exposed to ultraviolet irradiation. The strips are then stored until hybridization assays are conducted.

Capture probes are able to bind to the amplified products of the *Giardia* genome. Amplified product is allowed to bind to the membrane through hybridization to the capture probes. Amplified product is then detected by enzyme activity. A preferred means for detection of amplified product is to biotinylate each primer so that the amplified product is able to bind a streptavidin-horseradish peroxidase complex. The complex then permits detection of amplified product by chromogen development using commercially available substrates. Primer pairs may be biotinylated using the procedures described in Levenson C. and C. Chang, Nonisotypically Labeled Probes and Primers in *PCR Protocols*, Ed. Innis et al., Academic Press Inc., pp. 99-112, 1990.

Amplified target DNA can also be detected by high pressure liquid chromatography [HPLC]. Separation of the amplified PCR target DNA product, side products, and unreacted reagents by HPLC can provide a rapid quantitative report on the presence or absence of amplified DNA of the expected size range. HPLC columns may, for example, be based on ion exchange, paired-ion reverse-phase, or size exclusion separations. The column effluent is generally most simply detected and quantified by ultraviolet absorbance in the 250-280 nm spectral region, although fluorescent monitoring, after post-column derivatization with a fluorescent DNA-binding dye, and electrochemical detection also are possible and generally are potentially more sensitive than spectrophotometry. Separation of amplified PCR target DNA product, side products, and unreacted reagents by gel electrophoresis, followed by DNA staining with a fluorescent or absorbing dye, also reports on the presence or absence of amplified DNA in the expected size range.

Table 3.  Preferred gene probe sequences for the capture or detection of target DNA gene sequences of the gene encoding *giardin*.


GGP105-145

    5'-AGCAGGTTCCACGACAAGATGGAGAACGAGATCGAGGTCCG-3' (SEQ ID NO 5) for PCR amplified product formed using primers GGL78-97 and GGR171-190 for genus *Giardia*


GGP318-342

    5'-ATGGCTGCAAACTTCCGCAAGTCCC-3' (SEQ ID NO 6) for PCR amplified product formed using primers GGL267-286 and GGR366-385 for genus *Giardia*


GGP751-776

    5'-TCGAGGACGTCGTCTCGAAGATCCAG-3' (SEQ ID NO 7) for PCR amplified product formed using primers GGL639-658 and GGR789-809 for genus *Giardia*


GGP510-537

    5'-AGCTCAACGAGAAGGTCGCAGAGGGCTT-3' (SEQ ID NO 8) for PCR amplified product formed using primers GGL405-433 and GGR592-622 for specific detection of *G. lamblia*


A further aspect of this invention comprises kits suitable for use in carrying out the PCR amplifications and detection processes of this invention. Such test kits, designed to facilitate the amplification and detection of a waterborne pathogen, will generally comprise a primer pair consisting of two oligonucleotide primers complementary to about 10-30 nucleotide sequences on complementary strands of a targeted RNA or DNA sequence in a target gene of said pathogen, and a probe sequence for detection of a targeted subsequence and optionally a control DNA template of said targeted subsequence. The test kits may further comprise published instructions and reagents for the PCR amplification and detection of the targeted DNA sequence.

Reagents for the PCR amplification of the targeted RNA or DNA subsequence would include: deoxyribonuclease, reverse transcriptase, PCR amplification polymerase and the like, and filtration devices for water sample collection.

The kits may also include a bulk polymerase chain amplification reaction mix which would contain optimized concentrations of a thermal resistant nucleic acid polymerase, select primers, dNTPs, IPC, and buffer salts.

Preferred test kits, according to this invention, comprise the aforementioned preferred primer pairs and the corresponding preferred probe sequences also mentioned hereinbefore; and, optionally, a control DNA template of the targeted DNA sequence. For example, a test kit for detection of genus *Giardia* may comprise the primer pair 5'-AAGCTCAGCAACATGAACCA-3' (SEQ ID NO 17) and 5'-ATGGCGTCCTTGATCATCTT-3' (SEQ ID NO 18) and the gene probe 5'-AGCAGGTTCCACGACAAGATGGAGAACGAGATCGAGGTCCG-3'(SEQ ID NO 5) or the complement of this sequence for amplification of a sequence in the *giardin* gene and for detection of an amplified sequence in said *giardin* gene. A test kit for detection of *G. lamblia* or *G. duodenalis* only, may comprise the primer pair 5'-CATAACGACGCCATCGCGGCTCTCAGGAA-3' (SEQ ID NO 23) and 5'-TTTGTGAGCGCTTCTGTCGTGGCAGCGCTAA-3' (SEQ ID NO 24) the gene probe 5'-AGCTCAACGAGAAGGTCGCAGAGGGCTT-3' (SEQ ID NO 8) or the complement of this sequence for amplification of a sequence in the *giardin* gene and for detection of an amplified sequence in said *giardin* gene.

The kit may also comprise a means to detect the amplified product. Although any standard detection means can be used, such as a hybridization dot blot or agarose acrylamide gel electrophoresis, a preferred method uses a dip stick having a capture oligonucleotide which binds specifically to the amplified target. The dip stick captures the target and is detected by a non-isotopic detection system. For example, the primers may be biotinylated before extension and a horseradish peroxidase/streptavidin complex used to bind the enzyme specifically to the capture site of the amplified target. A suitable substrate\buffer combination is 3, 3', 5, 5' tetra methylbenzidine (TMB) in 100 mM Na Citrate at pH 5.0.

As can be readily envisioned the reaction reagents for amplifying the subsequences of the gene encoding *giardin* can be in separate compartments or combined in an amplification tube. For example a tube might contain the amplification mixture, specific primers and Taq polymerase. Before use, the tube is merely opened and the remaining reagents added including the test sample.

The invention is illustrated by the following exemplary tests, and the results thereof, for the recovery, PCR amplification and detection of waterborne *Giardia*. It will be apparent to those of skill that various substitutions and modifications may be made to the invention disclosed herein without departing from the scope and spirit of the invention. The following examples are provided for illustration purposes and should not be construed as a limitation of this invention.

Examples

A. General methods.

1. Three methods are useful for recovering DNA from *Giardia* cells. In the first method, total genomic DNA is extracted from trophozoites by first washing the cells in cold phosphate buffered saline [PBS] buffer of 0.145 M NaCl, 0.76 mM $NaH_2PO_4 \cdot H_2O$, 2.24 mM $Na_2HPO_4 \cdot 7H_2O$ at pH 7.2 with HCl released by alkaline lysis with sodium dodecyl sulfate (SDS) treatment. (See Ausubel, F.M. et al. eds., *Current Protocols in Molecular Biology*, John Wiley & Sons, Inc. NY, 1987). Proteinase K (Sigma, St. Louis, USA) was used to remove proteins and the DNA was further purified by using chloroform:isoamyl alcohol (24:1) and phenol:chloroform:isoamyl alcohol (24:24:2) extractions followed by precipitation with isopropanol. After centrifugation at 12,000 x g for 15 min, the pelleted DNA was washed once with cold 70% alcohol and dried under vacuum.

In two simpler lysis methods of recovery of target DNA from *Giardia* cysts, cysts were collected by centrifugation at 16,000 x g for 5 min, resuspended in 50 $\mu$l 0.1% DEPC in water and either heated to 90°C for 5 min followed by quick cooling in ice or at 5°C for 2 mins, or were lysed by freeze-thaw cycling.

2. PCR amplification was performed using a DNA Thermal Cycler and *Taq* polymerase. The PCR solution contained 1 x PCR amplification buffer (10 x buffer contains 50 mM KCl, 100 mM Tris•Cl, pH 8.13, 15 mM MgCl and 0.1% (w/v) gelatin), 200 $\mu$M each of the dNTPs, 0.2-1 $\mu$M2 of each of the primers, 1 ag (10-18 g) - 1 $\mu$g template DNA, 2.5 units *Taq* DNA polymerase, and double distilled water containing 0.1% DEPC. Template target DNAs were initially denatured at 94°C for 1-3 min. Then a total of 25-40 PCR cycles were run using the following conditions: denaturation at 94°C for 1 min, primer annealing and extension at 50°C or 60°C for 1 min.

Oligonucleotide primers were synthesized using a DNA synthesizer and purified using an oligonucleotide Purification Cartridge (Applied Biosystems, Foster City, USA) for small samples and reverse-phase HPLC with a C-8 3 micron reverse-phase column for large samples.

The first region is a 113 bp region of *giardin* gene amplified by using 20 mer primers GGL78-97, 5'-AAGCTCAGCAACATGAACCA-3' (SEQ ID NO 17) and GGR171-190, 5'-ATGGCGTCCTTGATCATCTT-3'-(SEQ ID NO 18). Primer GGL78-97 was located between 78 bp and 97 bp and primer GGR171-190 was located between 171 bp and 190 bp within the coding sequence of the *giardin* gene.

The second region of the *giardin* gene sequence, 119 bp long, was amplified using primers GGL267-286, 5'-AAGAAGTCCGCCGACAACAT-3' (SEQ ID NO. 19) and GGR336-385, 5'-AGATTTGTCTCAACGTTGTT-3' (SEQ ID NO 20). Primer GGL267-286 is located between 267 bp and 286 bp and primer GGR366-385 is located between 366 bp and 385 bp within the coding sequence of the *giardin* gene.

The third region of the *giardin* gene sequence, 218 bp long, was amplified by primers GGL405-433, 5'-CATAACGACGCCATCGCGGCTCTCAGGAA-3' and GGR492-622, 5'-TTTGTGAGCGCTTCTGTCGTGGCAGCGCTAA-3'. Primer GGL405-433 is located between 405 bp and 433 bp and primer GGR592-622 is located between 592 bp and 622 bp within the coding sequence of the *giardin* gene.

The fourth region of the *giardin* gene sequence, 171 bp long, was amplified by using primers GGL639-658, 5'-AAGTGCGTCAACGAGCAGCT-3' and GGR789-809, 5'-TTAGTGCTTTGTGACCATCGA-3'. Primer GGL639-658 is located between 639 bp and 658 bp and primer GGR789-809 is located between 789 bp and 809 bp within the coding sequence of the *giardin* gene.

In examples utilizing multiplex PCR, where two regions of the *giardin* gene are amplified simultaneously using mixtures of two primer sets, one primer set was GGL405-433 and GGR592-622 and the other primers set was any one of the other three sets described above. Equimolar quantities of the two primer sets were utilized.

3. PCR amplified targeted *Giardia* DNA sequences are detected by using gel electrophoresis and radiolabeled gene probes. The amplified target DNA references are separated using either 0.8-1% horizontal agarose gels or 10% vertical polyacrylamide gels. Agarose gels are run in TAE buffer (0.04 M Tris-acetate and 0.001 M EDTA, pH 8.0). Polyacrylamide gels are run in TBE buffer (0.89 M Tris-borate, 0.089 M boric acid and 0.002 M EDTA, pH 8.0) at 5.7-9.0 V/cm for 2-4 h. The gels are stained in 2 x 10-4% ethidium bromide solution and visualized with a Photo/PrepI UV transilluminator (Fotodyne Inc., New Berlin, USA) and photographed.

For Southern blots the amplified target DNA sequences are transferred onto nylon membranes (ICN Biomedicals, Costa Mesa, USA or Bio-Rad, Richmond, USA) using 0.4 M NaOH solution and fixed onto the membranes either by baking for 1 h at 80°C or by UV irradiation. For dot blots, the double-stranded amplified target DNA sequences are denatured by adding a denaturing solution containing 0.1 volume 3 M NaOH and 0.1 M Na$_2$EDTA, incubated at 60°C for 15 min-1 h, and neutralized with 1 volume cold 2 M ammonium acetate; the samples are then spotted onto Zeta probe nylon membranes (Bio-Rad) using a (Bio-Rad) dot blot manifold at a 4-5 psi vacuum pressure.

The amplified DNAs immobilized on the ICN nylon membranes were prehybridized with a hybridization solution containing 5 x SSPE (1 x SSPE is 10 mM sodium phosphate, pH 7.0, 0.18 m NaCl, 1 mM Na$_2$EDTA), 0.5% Denhardt's solution, and 100 $\mu$g per ml phenol extracted, denatured, salmon sperm DNA (Sigma) or 50 $\mu$g per ml type X Baker's yeast tRNA (Sigma); prehybridization was at 55-60°C for 3-16 h. The blots are washed twice in 2 x SSPE, 0.5% at room temperature for 10 min each and once in 0.1 x SSPE, 0.1% SDS at 55°C for 3-5 min with gently agitation. To detect $^{32}$P-labelled DNAs, the blots are covered with saran wrap (Fisher Biochemical, Pittsburgh, USA) and x-ray film (is placed over them; film exposure is at -70°C for 1-48 h.

The preferred probes for use in this invention are provided in Table 3. They include the 41 mer gene probe GGP105-145, which is used for detection of *giardin* DNA amplified using primers GGL78-97 and GGR171-190; the 25 mer gene probe GGP318-342, which is used for detection of *giardin* DNA amplified using primers GGL267-286 and GGR366-385; the 28 mer gene probe GGP510-537, 5'-AGCTCAACGAGAAGGTCGCAGAGGGCTT-3', which is used for detection of *giardin* DNA amplified using primers GGL405-433 and GGR592-622; and, the 26 mer gene probe GGP751-776, which is used for detection of *giardin* DNA amplified using primers GGL639-658 and GGR789-809.

All gene probes hybridize to target sequences located within the respective regions of amplified target DNA sequences. The gene probes were 5-end radiolabelled with [32P]ATP (>3000 Ci/mmol) by a procedure in which a 30 $\mu$l reaction solution contained 50 mM Tris•HCl, pH 7.5, 10 mM MgCl$_2$, 5 mM DTT, 1 mM KCl, 1-10 $\mu$g oligonucleotide gene probe, 120 pmol [32P]ATP (specific activity >3000 Ci/mmol), 1 mM spermidine (disodium salt), and 20 units of T4 polynucleotide kinase. The reaction mixture was incubated at 37°C for 1 h and the radiolabelled probes were purified by using a Sephadex G-50 column and TE buffer (10 mM Tris•Cl, pH 7.6, 1 mM Na$_2$EDTA).

B. Specificity of *Giardia* Detection.

To illustrate the specificity of *Giardia* detection by PCR amplification tests were conducted using the four primer sets described above and protozoan and nonprotozoan DNAs listed in Table 4.

Table 4

| Giardia lamblia vis-CDC | Trichomonas vaginalis 30001 |
|---|---|
| G. lamblia H-1-P | Eimeria nieschulzi |
| G. lamblia H-2-P | Chlorella |
| G. lamblia WB(FG) | Scenedesmus |
| G. lamblia MR-4 | Chlorococcum |
| G. lamblia Be-1 | Escherichia coli |
| G. muris | Legionella pneumonhila |
| G. intestinalis 50137 | Salmonella typhimurium |
| G. intestinalis 50114 | Shigella |
| Acanthamoeba castellais 30011 | Pseudomonas cepacia |
| Entamoeba histolvtica HM-1 | P. diminuta |
| Entamoeba histolytica 30015 | P. fluorescens |
| Cryptosporidium parvum | P. aeroginosa |
| C. muris | Alcaligenes faecalis |
| Dientamoeba fragilis 40948 | Saccharomyces cerevisiae |
| Entamoeba coli 30946 | Ustilago violacea |
| Blastodium hominis 50177 | Human DNA |

DNAs were extracted from cultures of these strains and 50ng to 1 $\mu$g of recovered DNAs subjected to PCR amplification as previously described using varying annealing temperatures during the PCR procedure; gene probes were used to detect amplified DNA sequences by both Southern and dot blot procedures as described hereinbefore. The specific activities of the radiolabelled probes were 68,000-167,000 DPM/$\mu$g DNA.

PCR amplification using primers GGL639-658 and GGR789-809 and a primer annealing temperature of 60°C produced amplified DNA bands for all *Giardia* strains tested. No non-*Giardia* species tested showed DNA amplification using the primers for the *giardin* gene (See Table 5). Southern blot hybridizations with gene probe GGP751-776 indicated that amplification of the target sequence was specific was *Giardia*.

Table 5

| | | |
|---|---|---|
| *Giardia lamblia* vis-CDC | + | + |
| *G. lamblia H-1-P* | + | + |
| *G. lamblia H-2-P* | + | + |
| *G. lamblia WB(FG)* | + | + |
| *G. lamblia MR-4* | + | + |
| *G. lamblia Be-1* | + | + |
| *G. muris* | + | - |
| *G. intestinalis 50137* | + | + |
| *G. intestinalis 50114* | + | + |
| Acanthamoeba castellais 30011 | - | - |
| Entamoeba histolytica HM-1 | - | - |
| Entamoeba histolytica 30015 | - | - |
| Cryptosporidium parvum | - | - |
| C. muris | - | - |
| Dientamoeba fragilis 40948 | - | - |
| Entamoeba coli 30946 | - | - |
| Blastodium hominis 50177 | - | - |
| Trichomonas vaginalis 30001 | - | - |
| Eimeria nieschulzi | - | - |
| Chlorella | - | - |
| Scenedesmus | - | - |
| Chlorococcum | - | - |
| Escherichia coli | - | - |
| Legionella pneumophila | - | - |
| Salmonella typhimurium | - | - |
| Shigella | - | - |
| Pseudomonas cepacia | - | - |
| P. diminuta | - | - |
| P. fluorescens | - | - |
| P. aeroginosa | - | - |
| Alcaligenes faecalis | - | - |
| Saccharomyces cerevisiae | - | - |
| Ustilago violacea | - | - |
| Human DNA | - | - |

C. Distinguishing between live and dead Giardia cysts usingRNA

1. *Giardia* cysts are collected by centrifugation at 16,000 g for 5 min and resuspended in 50 μl water containing 0.1% DEPC. Cells were heated to 90°C for 5 min followed by cooling at 5°C for 2 min or in ice. Ten microliters of 10x PCR buffer (described above) is added, along with 1 μl RNasin and 1 μl deoxyribonuclease (DNase) and incubated at 37°C for 60 min. The DNase is inactivated at 99°C for 5 min followed by cooling at 5°C for 2 min or in ice.

RNA was extracted from dead cysts (killed by freezing), from live uninduced cysts, and from live induced cysts, using the RNAgents total RNA isolation kit from Promega, USA. The concentration of RNA was measured by spectroscopy.

2. cDNA was synthesized from undegraded mRNA using MMLV reverse transcriptase. After DNase inactivation 200 μM each of the dNTPs, 1 μM of primer GGR789-809 and 1 μl MMLV reverse transcriptase were added and the reaction mixture was incubated at 42°C for 30 min followed by inactivation of the enzyme at 99°C for 5 min and cooling at 5°C for 5 min or in ice.

3. Primer GGL639-658 (1 μM) was added, along with 1 μl *Taq* polymerase and subjected to 35 cycles of PCR amplification. The parameters of the amplification process were initial time delay 95°C for 2 min, denaturation 95°C for 1 min, reannealing and extension at 60°C for 1 min and final extension at 60°C for 7 min.

4. PCR amplification after DNase treatment of lysed cysts followed by cDNA synthesis produced amplified DNA bands, indicating the presence of mRNA in the cyst. RNA extracted from dead cysts did not show amplification thus distinguishing live cysts from dead cysts. While DNA-PCR shows amplification for both dead and live cysts, RNA-PCR amplifies only live cysts. Results of these experiments are provided in Table 6.

Table 6

| | RNA yield from $10^6$ cysts | DNA PCR | RNA PCR |
|---|---|---|---|
| Dead cysts | 1 $\mu$g | + | - |
| Uninduced live cysts | 2 $\mu$g | + | + |
| Stage 1 of induction: Live cysts incubated with reducing solution and NaHCO3 37°C for 30 mins | 16 $\mu$g | + | + |
| Stage 2 of induction: Live cysts incubated as above and washed once in trypsin-tyrodes solution | 125 $\mu$g | + | + |
| Stage 3 of induction: Live cysts treated as above followed by incubation in trypsin-tyrodes solution at 37°C for 30 mins | 127 $\mu$g | + | + |

D. Sensitivity of *Giardia* Detection by PCR Amplification

To illustrate the sensitivity of PCR amplification-gene probe detection of *Giardia*, single cysts were separated out under a Zeiss Axiovert 35 inverted microscope using a de Fonbrune pneumatic micromanipulator. The cysts were heated to 90°C for 5 min, cooled to 5°C for 2 min and subjected to PCR amplification using primers GGL639-658 and GGR789-809 (Table 1). Amplified DNA was analyzed by polyacrylamide gel electrophoresis and Southern blot analysis using gene probe GGP751-776 (Table 3).

In 15 trials, the primers were repeatably able to detect the single cyst.

E. Amplification of cDNA from *G. muris* cysts

*G. muris* cysts were induced to three progressive stages of induction and excystation. In stage 1 cysts were induced (but not allowed to excyst) by incubation in reducing solution (0.1 g Glutathione, 0.1 g L-cysteine-HCl in 10 ml 1x Hank's balanced salt solution) and 0.1M sodium bicarbonate at 37°C for 30 min. In stage 2 cysts were induced as in stage 1 and washed once in trypsin-tyrode's solution (0.5 g trypsin, 0.15 g NaHCO₃ in 100 ml 1x tyrode's solution). 10x tyrodes solution contains 160 g NaCl 4 g KCl, 4 g CaCl₂, 2 g MgCl2 • 6H2O, 1 g NaH2PO4 • H2O and 20 g glucose in 1 l $H_2O$. In stage 3 cysts were treated as in stage 2 and excysted by incubation in trypsin-tyrode's solution at 37°C for 30 min.

*G. muris* DNA-free RNA extracted from dead cysts, live uninduced cysts and live cysts induced to three different stages, showed an increase in yield of RNA after induction, which reached a plateau at stage 2 of induction (Table 6). RNA PCR was performed on these RNAs using primers GGL639-658 and GGR789-809 (Table 1). Amplification was seen only with live induced and uninduced cysts, and not with dead cyst RNA.

SEQUENCE LISTING

(2) INFORMATION FOR SEQ ID NO:1:

    (i) SEQUENCE CHARACTERISTICS:
        (A) LENGTH: 41 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..41
        (D) OTHER INFORMATION: /function= "Probe Binding Site"
            /label= GGP105-145

    (xi) SEQUENCE DESCRIPTION: SEQ ID NO:1:

CGGACCTCGA TCTCGTTCTC CATCTTGTCG TGGAACCTGC T                  41

(2) INFORMATION FOR SEQ ID NO:2:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..25
        (D) OTHER INFORMATION: /function= "Probe Binding Site"
            /label= GGP318-342

    (xi) SEQUENCE DESCRIPTION: SEQ ID NO:2:

GGGACTTGCG GAAGTTTGCA GCCAT                        25

(2) INFORMATION FOR SEQ ID NO:3:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..26
        (D) OTHER INFORMATION: /function= "Probe Binding Site"
            /label= GGP751-776

    (xi) SEQUENCE DESCRIPTION: SEQ ID NO:3:

CTGGATCTTC GAGACGACGT CCTCGA                          26


(2) INFORMATION FOR SEQ ID NO:4:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..28
        (D) OTHER INFORMATION: /function= "Probe Binding Site"
            /label= GGP510-537

    (xi) SEQUENCE DESCRIPTION: SEQ ID NO:4:

AAGCCCTCTG CGACCTTCTC GTTGAGCT                        28

(2) INFORMATION FOR SEQ ID NO:5:

    (i) SEQUENCE CHARACTERISTICS:
        (A) LENGTH: 41 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..41
        (D) OTHER INFORMATION: /function= "probe"
            /label= GGP105-145

    (xi) SEQUENCE DESCRIPTION: SEQ ID NO:5:

AGCAGGTTCC ACGACAAGAT GGAGAACGAG ATCGAGGTCC G                    4:


(2) INFORMATION FOR SEQ ID NO:6:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..25
        (D) OTHER INFORMATION: /function= "Probe"
            /label= GGP318-342

    (xi) SEQUENCE DESCRIPTION: SEQ ID NO:6:

ATGGCTGCAA ACTTCCGCAA GTCCC                       25

(2) INFORMATION FOR SEQ ID NO:7:

    (i) SEQUENCE CHARACTERISTICS:
        (A) LENGTH: 26 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..26
        (D) OTHER INFORMATION: /function= "Probe"
            /label= GGP751-776

    (xi) SEQUENCE DESCRIPTION: SEQ ID NO:7:

TCGAGGACGT CGTCTCGAAG ATCCAG                       26


(2) INFORMATION FOR SEQ ID NO:8:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..28
        (D) OTHER INFORMATION: /function= "Probe"
            /label= GGP510-537

    (xi) SEQUENCE DESCRIPTION: SEQ ID NO:8:

AGCTCAACGA GAAGGTCGCA GAGGGCTT                     28

(2) INFORMATION FOR SEQ ID NO:9:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer Binding Region"
            /label= GGL78-97

    (xi) SEQUENCE DESCRIPTION: SEQ ID NO:9 :

TGGTTCATGT TGCTGAGCTT                                 20

(2) INFORMATION FOR SEQ ID NO:10:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer Binding Region"
            /label= GGR171-190

    (xi) SEQUENCE DESCRIPTION: SEQ ID NO:10:

AAGATGATCA AGGACGCCAT                                 20

(2) INFORMATION FOR SEQ ID NO:11:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer Binding Region"
            /label= GGL267-286

    (xi) SEQUENCE DESCRIPTION: SEQ ID NO:11:

ATGTTGTCGG CGGACTTCTT                    20


(2) INFORMATION FOR SEQ ID NO:12:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer Binding Region"
            /label= GGR366-385

    (xi) SEQUENCE DESCRIPTION: SEQ ID NO:12:

AACAACGTTG AGACAAATCT                    2C

(2) INFORMATION FOR SEQ ID NO:13:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer Binding Region"
            /label= GGL639-658

    (xi) SEQUENCE DESCRIPTION: SEQ ID NO:13:

AGCTGCTCGT TGACGCACTT                                 20


(2) INFORMATION FOR SEQ ID NO:14:

    (i) SEQUENCE CHARACTERISTICS:
        (A) LENGTH: 21 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..21
        (D) OTHER INFORMATION: /function= "Primer Binding Region"
            /label= GGR789-809

    (xi) SEQUENCE DESCRIPTION: SEQ ID NO:14:

TCGATGGTCA CAAAGCACTA A                               21

(2) INFORMATION FOR SEQ ID NO:15:

    (i) SEQUENCE CHARACTERISTICS:
        (A) LENGTH: 29 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..29
        (D) OTHER INFORMATION: /function= "Primer Binding Region"
            /label= GGL405-433

    (xi) SEQUENCE DESCRIPTION: SEQ ID NO:15:


TTCCTGAGAG CCGCGATGGC GTCGTTATG                  29


(2) INFORMATION FOR SEQ ID NO:16:

    (i) SEQUENCE CHARACTERISTICS:
        (A) LENGTH: 31 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..31
        (D) OTHER INFORMATION: /function= "Primer Binding Region"
            /label= GGR592-622

    (xi) SEQUENCE DESCRIPTION: SEQ ID NO:16:

TTAGCGCTGC CACGACAGAA GCGCTCACAA A               31

(2) INFORMATION FOR SEQ ID NO:17:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer"
            /label= GGL78-97

    (xi) SEQUENCE DESCRIPTION: SEQ ID NO:17:

AAGCTCAGCA ACATGAACCA                                                              2(

(2) INFORMATION FOR SEQ ID NO:18:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..20
        (D) OTHER INFORMATION: /function= "Primer"
            /label= GGR171-190

    (xi) SEQUENCE DESCRIPTION: SEQ ID NO:18:

ATGGCGTCCT TGATCATCTT                                                              2C

(2) INFORMATION FOR SEQ ID NO:19:

    (i) SEQUENCE CHARACTERISTICS:
       (A) LENGTH: 20 base pairs
       (B) TYPE: nucleic acid
       (C) STRANDEDNESS: single
       (D) TOPOLOGY: linear

    (ii) MOLECULE TYPE: DNA

    (ix) FEATURE:
       (A) NAME/KEY: misc_feature
       (B) LOCATION: 1..20
       (D) OTHER INFORMATION: /function= "Primer"
           /label= GGL267-286

    (xi) SEQUENCE DESCRIPTION: SEQ ID NO:19:

AAGAAGTCCG CCGACAACAT                        20


(2) INFORMATION FOR SEQ ID NO:20:

    (i) SEQUENCE CHARACTERISTICS:
       (A) LENGTH: 20 base pairs
       (B) TYPE: nucleic acid
       (C) STRANDEDNESS: single
       (D) TOPOLOGY: linear

    (ii) MOLECULE TYPE: DNA

    (ix) FEATURE:
       (A) NAME/KEY: misc_feature
       (B) LOCATION: 1..20
       (D) OTHER INFORMATION: /function= "Primer"
           /label= GGR366-385

    (xi) SEQUENCE DESCRIPTION: SEQ ID NO:20:

AGATTTGTCT CAACGTTGTT                        20


(2) INFORMATION FOR SEQ ID NO:21:

    (i) SEQUENCE CHARACTERISTICS:
       (A) LENGTH: 20 base pairs
       (B) TYPE: nucleic acid
       (C) STRANDEDNESS: single
       (D) TOPOLOGY: linear

    (ii) MOLECULE TYPE: DNA

    (ix) FEATURE:
       (A) NAME/KEY: misc_feature
       (B) LOCATION: 1..20
       (D) OTHER INFORMATION: /function= "Primer"
           /label= GGL639-658

    (xi) SEQUENCE DESCRIPTION: SEQ ID NO:21:

AAGTGCGTCA ACGAGCAGCT                        20

(2) INFORMATION FOR SEQ ID NO:22:

    (i) SEQUENCE CHARACTERISTICS:
        (A) LENGTH: 21 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..21
        (D) OTHER INFORMATION: /function= "Primer"
           /label= GGR789-809

    (xi) SEQUENCE DESCRIPTION: SEQ ID NO:22:

TTAGTGCTTT GTGACCATCG A              21


(2) INFORMATION FOR SEQ ID NO:23:

    (i) SEQUENCE CHARACTERISTICS:
        (A) LENGTH: 29 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..29
        (D) OTHER INFORMATION: /function= "Primer"
           /label= GGL405-433

    (xi) SEQUENCE DESCRIPTION: SEQ ID NO:23:

CATAACGACG CCATCGCGGC TCTCAGGAA           29


(2) INFORMATION FOR SEQ ID NO:24:

    (i) SEQUENCE CHARACTERISTICS:
        (A) LENGTH: 31 base pairs
        (B) TYPE: nucleic acid
        (C) STRANDEDNESS: single
        (D) TOPOLOGY: linear

    (ii) MOLECULE TYPE: DNA

    (ix) FEATURE:
        (A) NAME/KEY: misc_feature
        (B) LOCATION: 1..31
        (D) OTHER INFORMATION: /function= "Primer"
           /label= GGR592-622

    (xi) SEQUENCE DESCRIPTION: SEQ ID NO:24:

TTTGTGAGCG CTTCTGTCGT GGCAGCGCTA A      31

(2) INFORMATION FOR SEQ ID NO:25:

    (i) SEQUENCE CHARACTERISTICS:
        (A) LENGTH: 88 base pairs
        (B) TYPE: nucleic acid
        (C) STRANDEDNESS: single
        (D) TOPOLOGY: linear

   (ii) MOLECULE TYPE: DNA

   (ix) FEATURE:
        (A) NAME/KEY: misc_feature
        (B) LOCATION: 1..88
        (D) OTHER INFORMATION: /note= "Upstream Primer Binding
           Site"

   (xi) SEQUENCE DESCRIPTION: SEQ ID NO:25:

TTTGATGGAG ATGAGGAGTT CTACGTGGAC CTGGAGAGGA AGGAGACTGC CTGGCGGTGG   60

CCTGAGTTCA GCAAATTTGG AGGTTTTG                         88


(2) INFORMATION FOR SEQ ID NO:26:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

   (ii) MOLECULE TYPE: DNA

   (ix) FEATURE:
        (A) NAME/KEY: misc_feature
        (B) LOCATION: 1..20
        (D) OTHER INFORMATION: /note= "Positive Control"

   (xi) SEQUENCE DESCRIPTION: SEQ ID NO:26:

TGAGTTCAGC AAATTTGGAG                             20

```
(2) INFORMATION FOR SEQ ID NO:27:

    (i) SEQUENCE CHARACTERISTICS:
        (A) LENGTH: 19 base pairs
        (B) TYPE: nucleic acid
        (C) STRANDEDNESS: single
        (D) TOPOLOGY: linear

    (ii) MOLECULE TYPE: DNA


    (ix) FEATURE:
        (A) NAME/KEY: misc_feature
        (B) LOCATION: 1..19
        (D) OTHER INFORMATION: /note= "Negative Control"


    (xi) SEQUENCE DESCRIPTION: SEQ ID NO:27:

GATGAGCAGT TCTACGTGG                                        19
```

## Claims

1. Method for detection of species of the genus *Giardia* having a copy of the gene encoding *giardin*, said method comprising:
   (a) hybridizing a nucleic acid probe to a complementary portion of the gene encoding *giardin*; and,
   (b) detecting the hybridization of the probe to the gene encoding *giardin*.

2. Method of claim 1 which further comprises before step (a) the step of amplifying a subsequence of the *giardin* gene.

3. Method of claim 2 wherein the amplifying is achieved by use the polymerase chain reaction method.

4. Method of any one of claims 1 to 3 wherein the probe binds to a conserved region of the gene encoding *giardin*.

5. Method of any one of claims 1 to 3 wherein the probe binds to a hypervariable region of the gene encoding *giardin*.

6. Method of any one of claims 1 to 3 wherein the probe binds substantially to a sequence selected from the group consisting of:

   (a) 3'-TCGTCCAAGGTGCTGTTCTACCTCTTGCTCTAGCTCCAGGC-5'
   (SEQ ID NO 1);

   (b) 3'-TACCGACGTTTGAAGGCGTTCAGGG-5'(SEQ ID NO 2); and

   (c) 3'-AGCTCCTGCAGCAGAGCTTCTAGGTC-5'(SEQ ID NO 3)

   or to the complement sequence thereof.

7. Method of any one of claims 1 to 3 wherein the probe binds substantially to a sequence consisting of:

   3'-TCGAGTTGCTCTTCCAGCGTCTCCCGAA-5' (SEQ ID NO 4)

   or to the complement sequence.

29

8. Method of any one of claims 1 to 3 wherein the probe comprises a nucleic acid subsequence selected from the group consisting of:

    (a)   5'-AGCAGGTTCCACGACAAGATGGAGAACGAGATCGAGGTCCG-3' (SEQ ID NO 5);

    (b)   5'-ATGGCTGCAAACTTCCGCAAGTCCC-3' (SEQ ID NO 6);

    (c)   5'-TCGAGGACGTCGTCTCGAAGATCCAG-3' (SEQ ID NO 7)

or the complement sequence thereof.

9. Method of any one of claims 1 to 3 wherein the probe comprises a nucleic acid subsequence consisting of:

5'-AGCTCAACGAGAAGGTCGCAGAGGGCTT-3' (SEQ ID NO 8)

or the complement sequence.

10. Recombinant nucleic acid hybridization probe comprising a subsequence consisting of:

    (a)   5'-AGCAGGTTCCACGACAAGATGGAGAACGAGATCGAGGTCCG-3' (SEQ ID NO 5);

    (b)   5'-ATGGCTGCAAACTTCCGCAAGTCCC-3' (SEQ ID NO 6);

    (c)   5'-TCGAGGACGTCGTCTCGAAGATCCAG-3' (SEQ ID NO 7);

and,

    (d)   5'-AGCTCAACGAGAAGGTCGCAGAGGGCTT-3' (SEQ ID NO 8)

or the complement sequence thereof.

11. Method of claim 3 wherein at least one of the primers binds substantially to a nucleic acid subsequence selected from the group consisting of:

    (a)  3'-TTCGAGTCGTTGTACTTGGT-5' (SEQ ID NO 9);

    (b)  3'-TACCGCAGGAACTAGTAGAA-5' (SEQ ID NO 10);

    (c)  3'-TTCTTCAGGCGGCTGTTGTA-5' (SEQ ID NO 11);

    (d)  3'-TCTAAACAGAGTTGCAACAA-5' (SEQ ID NO 12);

    (e)  3'-TTCACGCAGTTGCTCGTCGA-5' (SEQ ID NO 13);

    (f)  3'-AATCACGAAACACTGGTAGCT-5' (SEQ ID NO 14).

12. Method of claim 3 wherein at least one of the primers binds substantially to a nucleic acid subsequence selected from the group consisting of:

(a) 3'-GTATTGCTGCGGTAGCGCCGAGAGTCCTT-5'; (SEQ ID NO 15); and

(b) 3'-AAACACTCGCGAAGACAGCACCGTCGCGATT-5' (SEQ ID NO 16).

13. Method of claim 11 wherein the primer is selected from the group consisting of:

    (a)   5'-AAGCTCAGCAACATGAACCA-3' (SEQ ID NO 17);

    (b)   5'-ATGGCGTCCTTGATCATCTT-3'(SEQ ID NO 18);

    (c)   5'AAGAAGTCCGCCGACAACAT-3' (SEQ ID NO 19);

    (d)   5'-AGATTTGTCTCAACGTTGTT-3' (SEQ ID NO 20);

    (e)   5'-AAGTGCGTCAACGAGCAGCT-3' (SEQ ID NO 21); and,

    (f)   5'-TTAGTGCTTTGTGACCATCGA-3' (SEQ ID NO 22).

14. A method of claim 12 wherein the primer is selected from the group consisting of:

(a) 5'-CATAACGACGCCATCGCGGCTCTCAGGAA-3' (SEQ ID NO 23); and

(b) 5'-TTTGTGAGCGCTTCTGTCGTGGCAGCGCTAA-3' (SEQ ID NO 24).

15. Nucleic acid polymerase primer for the amplification of subsequences of nucleic acid from *Giardia* species wherein the primer binds substantially to a nucleic acid subsequence selected from the group consisting of:

    (a)   3'-TTCGAGTCGTTGTACTTGGT-5'; (SEQ ID NO 9);

    (b)   3'-TACCGCAGGAACTAGTAGAA-5' (SEQ ID NO 10);

    (c)   3'-TTCTTCAGGCGGCTGTTGTA-5' (SEQ ID NO 11);

    (d)   3'-TCTAAACAGAGTTGCAACAA-5' (SEQ ID NO 12);

    (e)   3'-TTCACGCAGTTGCTCGTCGA-5' (SEQ ID NO 13);

    (f)   3'-AATCACGAAACACTGGTAGCT-5' (SEQ ID NO 14).

16. Nucleic acid polymerase primer for the amplification of subsequences of nucleic acid from *Giardia* species wherein the primer binds substantially to a nucleic acid subsequence selected from the group consisting of:

   (a)  3'-GTATTGCTGCGGTAGCGCCGAGAGTCCTT-5';   (SEQ ID NO 15); and

   (b)  3'-AAACACTCGCGAAGACAGCACCGTCGCGATT-5'  (SEQ ID NO 16).

17. Nucleic acid polymerase primer of claim 15 wherein the primer is selected from the group consisting of:

   (a)   5'-AAGCTCAGCAACATGAACCA-3' (SEQ ID NO 17);

   (b)   5'-ATGGCGTCCTTGATCATCTT-3' SEQ ID NO 18);

   (c)   5'AAGAAGTCCGCCGACAACAT-3' (SEQ ID NO 19);

   (d)   5'-AGATTTGTCTCAACGTTGTT-3' (SEQ ID NO 20);

   (e)   5'-AAGTGCGTCAACGAGCAGCT-3' (SEQ ID NO 21); and

   (f)   5'-TTAGTGCTTTGTGACCATCGA-3' (SEQ ID NO 22).

18. A nucleic acid polymerase primer of claim 16 wherein the primer is selected from the group consisting of:

   (a)   5'-CATAACGACGCCATCGCGGCTCTCAGGAA-3' (SEQ ID NO 23); and,

   (b)   5'-TTTGTGAGCGCTTCTGTCGTGGCAGCGCTAA-3' (SEQ ID NO 24).

19. Kit for the detection of nucleic acid specific for *Giardia* species comprising a compartment containing at least one nucleic acid probe according to claim 8 and/or 9.

20. Kit of claim 19 which further comprises a compartment containing at least one polymerase chain reaction amplification primer according to claim 17 and/or 18.

21. Method of detecting live organisms from dead organisms in a sample suspected of containing living organisms, said method comprises:
    (a) treating the sample with sufficient heat to induce transcription of genes encoding proteins not otherwise transcribed;
    (b) lysing the cysts to release mRNA;
    (c) reverse transcribing the mRNA to cDNA;
    (d) amplifying target subsequences of the mRNA encoding the heat-induced genes; and
    (e) detecting the amplified target subsequences.

22. Method of claim 21 wherein the genes are selected from the group consisting of heat shock genes and *giardin*.

23. Method of claim 21 or 22 wherein the live organisms are cysts originating from species of *Giardia* and further comprises:
    (a) treating the cysts to heat sufficient to induce transcription of the gene encoding *giardin*;

32

(b) lysing the cysts to release mRNA;

(c) reverse transcribing the mRNA to cDNA;

(d) amplifying target subsequences of the mRNA encoding the *giardin* gene; and

(e) detecting the amplified target subsequences.

24. Method of any one of claims 21 to 23 wherein the amplifying in performed by the method according to claim 3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | ABSTRACTS GENERAL MEETING OF THE AMERICAN SOCIETY OF MICROBIOLOGY 91 vol. 0, 5 May 1991, DALLAS, US. page 85; MAHBUBANI, M. ET AL.: 'DETECTION OF VIABLE LEGIONELLA AND GIARDIA USING PCR AND GENE PROBES.' * abstract * | 1-3, 21-24 | C12Q1/68 C12P19/34 C07H21/04 |
| A | ABSTRACTS OF THE GENERAL MEETING OF THE AMERICAN SOCIETY OF MICROBIOLOGY 91 vol. 0, 5 May 1991, DALLAS, US. page 48; WEISS, J.B. ET AL.: 'SPECIFIC DETECTION OF GIARDIA LAMBLIA (G. DUODENALIS) BY THE POLYMERASE CHAIN REACTION.' * abstract * | 1-3 | |
| A | JOURNAL OF CELL BIOLOGY vol. 109, no. 5, November 1989, NEW YORK, US. pages 2323 - 2335; PEATTIE, D.A. ET AL.: 'ULTRASTRUCTURAL LOCALIZATION OF GIARDINS TO THE EDGES OF DISC MICRORIBBONS OF GIARDIA LAMBLIA.' | - | TECHNICAL FIELDS SEARCHED (Int. Cl.5) C12Q |
| A | APPLIED AND ENVIRONMENTAL MICROBIOLOGY vol. 57, no. 4, April 1991, WASHINGTON, DC. US. pages 927 - 931; ABBASZADEGAN, M. ET AL.: 'DETECTION OF GIARDIA CYSTS WITH A cDNA PROBE AND APPLICATIONS TO WATER SAMPLES' | - | |

The present search report has been drawn up for all claims

| Place of search | Date of completion of the search | Examiner |
|---|---|---|
| THE HAGUE | 25 AUGUST 1992 | OSBORNE H.H. |